# EUROPEAN PATENT SPECIFICATION

(11) **EP 1 495 019 B1**
(45) Date of publication and mention of the grant of the patent: **02.07.2008**
(21) Application number: 02714415.3
(22) Date of filing: 08.04.2002
(51) Int. Cl.: C07D 407/04, C07H 7/06, A61K 31/351, A61P 3/10

(54) **GLUCOPYRANOSIDE, PROCESS FOR ISOLATION THEREOF, PHARMACEUTICAL COMPOSITION CONTAINING SAME AND USE THEREOF**
GLUCOPYRANOSID, VERFAHREN ZU DEREN ISOLIERUNG, PHARMAZEUTISCHE ZUSAMMENSETZUNG UND VERWENDUNG DAVON
GLUCOPYRANOSIDE, SON PROCEDE D'ISOLATION, COMPOSITION PHARMACEUTIQUE CONTENANT LEDIT GLUCOPYRANOSIDE

(43) Date of publication of application: 12.01.2005
(73) Proprietor: COUNCIL OF SCIENTIFIC AND INDUSTRIAL RESEARCH, New Delhi 110 001 (IN)
(72) Inventor: MAURYA, Rakesh, Jammu (IN); SINGH, Deepa, Jammu (IN); BHAGAT, Asha, Jammu (IN); GUPTA, Om, Parkash, Jammu (IN); HANDA, Sukhdev, Swami, Jammu (IN)
(74) Representative: Texier, Christian
(86) International application number: PCT/IN2002/000105
(87) International publication number: WO 2003/087093

(56) References cited:
- GB-A- 2 359 554
- BEZUIDENHOUDT B C B ET AL: "FLAVONOID ANALOGUES FROM PTEROCARPUS SPECIES" PHYTOCHEMISTRY, PERGAMON PRESS, GB, vol. 26, no. 2, 1987, pages 531-535, XP008005725 ISSN: 0031-9422 cited in the application

## Description

### Field of the invention

The present invention relates to a novel glucopyranoside, 6-hydroxy-2-*p-*hydroxybenzylbenzofuran-7-C-β-D-glucopyranoside of the formula **1**

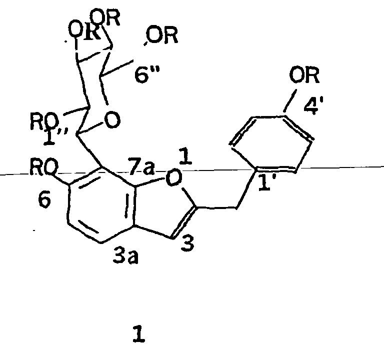

The present invention also relates to a process for the isolation of said novel glucopyranoside of formula 1 from *Pterocarpus marsupium.*

More particularly, the present invention relates to a process of isolation of 6-hydroxy-2-*p*-hydroxyhenzylbenzofuran-7-C-β-D-glucopyranoside of formula 1, from *Pterocarpus marsupium.* The present invention also relates to a pharmaceutical composition containing 6-hydroxy-2-*p*-hydroxybenzylbenzofuran-7-C-β-D-glucopyranoside of the formula **1** and to method for the treatment of diabetes using said compound of formula **1**.

GB2359,554 describes different C-glycosides showing a hypoglycemic activity and pharmaceutical compositions containing them for the treatment and prevention of diabetes.

### Backgroud of the invention

*Pterocarpus marsupium* Roxb (*Leguminosae*) also known as Indian Kino tree or Bijasar, is common in the hilly regions of central and peninsular India [Jain, S. K., Medicinal Plants, National Book Trust, New Delhi, 1968, p. 116]. The extracts of leaves, flowers and gum of this tree have been used medicinally in the treatment of diarrhea, toothache, fever, urinary tract and skin infections. [Chopra, R. N., Chopra, I. C., Handa, K. L. and Kapur, L. D., Indigenous Drugs of India, 2nd Ed., Dhar, U. N. and Sons Private Limited, Calcutta, 1958, p. 522]. The extract of the bark has long been regarded as useful in the therapy of diabetes [Kirtikar, K. R. and Basu, B. D., Indian Medicinal Plants, 2nd Ed., edited by Blatter, E., Cailes, J. F. and Mhaskar, K. S., Singh and Singh, Delhi, India, 1975, p. 2135]. It is reported by Chakravarthy et al [Chakravarthy, B. K., Gupta, S. and Gode, K. D., Lancet, 1982, 272 (and references cited therein)] that the active hypoglycemic principle of the bark is (-)-epicatechin and that its effect is due to the regeneration of pancreatic beta cells. However, this claim has been questioned by Kolb et al [Kolb, H., Kiesel, U., Grenlich, B. and Bosch, J. V. D., Lancet, 1982, 1303.] and Sheehan et al [Sheehan, E. W., Zemaitis, M. A., Slatkin, D. J. and Schiff, Jr., P. L., Journal of Natural Products, 1983, 46, 232]. It is now felt that further investigation is necessary before (-)-epicatechin can be considered a viable antidiabetic agent for use in human clinical studies.

Practitioners of the Indian System of Medicine are of the view that the heartwood rather than the bark of *Pterocarpus marsupium* is useful for treatment of diabetic patients and that older the plant more efficacious is its heartwood. It is also claimed that only heartwood that is distinctly red in colour and which imparts a red colouration with bluish green fluorescence to water in which it is kept soaked is suitable for use as an antidiabetic drug. Hypoglycaemic effects of aqueous or alcoholic extracts of heartwood of *Pterocarpus marsupium* have been verified by experimental [Shah, D. S., Indian Journal of Medical Research, 1967, 55, 166 and references cited therein; Gupta, S. S., Indian Journal of Medical Research, 1963, 51, 716] and clinical studies [Sepha, G. C. and Bose, S. N., J. Ind. Med. Assoc., 1956, 27, 383; Kedar, P. and Chakrabarti, C. H., Maharashtra Med. J., 1981, 28, 165]. The heartwood of *Pterocarpus marsupium* is rich in phenolics. Chemical investigation on heartwood of *P. marsupium* dates back to 1946 but early works [Bhargava, P. N., Proc. Ind. Acad. Sci., 1946, 24A, 496] on this drug are fragmentary in nature. Previous reported studies on this plant disclose the following chemical constituents.
1. The ether extract of *P. marsupium* heartwood furnished isoflavonoid glycol 4,4'-dihydroxy-α-methylhydrobenzoin designated Marsupol [Rao, A. V. S., Mathew, J., Phytochemistry, 1982, 21, 1837], a benzofurannone derivative, 2,4',6-trihydroxy-4-methoxybenzo(b)furan-3(2*H*)-one designated carpusin [Mathew, J. and Rao, A. V. S., Phytochemistry, 1983, 22, 794], 2-propanol derivative, 1,3-bis (4-hydroxyphenyl)propan-2-ol, designated propterol [Rao, A. V. S., Mathew, J. and Shankaran, A. V. B., Phytochemistry, 1984, 23, 897], 1-(2,4-dihydroxyphenyl)-3-(4-hydroxyphenyl)propan-2-ol designated propterol B [Mathew, J., Rao, A. V. S. and Rambhav, S. Current Science, 1984, 53, 576], 6-hydroxy-7-O-methyl-3-(3-hydroxy-4-O-methyl benzyl) chroman-4-one [Jain, S. C., Sharma, S. K., Kumar, R, Rajwansh, V. K. and Babu, V. R., Phytochemistry, 1997, 44, 765].
2. Ethyl acetate soluble fraction of alcoholic extract of the heartwood furnished pterosupin β, 2', 4,4'-tetrahydroxy-3'(c-β-D-glucopyranoside)dihydrochalcone [Adinarayana, D., Syamsundar, K. V., Seligmann, O., and wagner, H., (Z.Naturforsch., 1982, 37C, 145)], Marsupinol [Trivedi, J. J., Indian J. Phys. Pharmacol, 1997, 15, 51], 5, 4'-dimethoxy-8-methylisoflavone - 7 - O - α - L - rhamnopyranoside, retusin - O - β - D glucopyranoside and irisolidine-7-O-α-L-rhamnopyranoside [Mitra, J. and Joshi, T., Phytochemistry, 1982, 21, 2429] and 5, 7'-dihydroxy-6-methoxy-7-O-α-L-rhamnopyranoside [Mitra, J. and Joshi, T., Phytochemistry, 1983, 22, 2326] obtained from the ethyl acetate soluble fraction of alcoholic extract of the heartwood.
3. Novel benzofuranone derivative, 2,6-dihydroxy-2-(*p*-hydroxybenzyl)-4-methoxy-3(2H)-benzofuranone designated marsupin [Maurya, R., Ray, A. B., Duah, F. K., Slatkin, D. J. and Schiff, P. L. Jr., Heterocycles, 1982, 19, 2103], pterostilbin, (2S)- hydroxyflavone, isoliquiritigenin, liquiritigenin, 7, 4'-dihydroxyflavone, 5-deoxykaempferol and 3,7,4'-trihydroxyflavone [Maurya, R., Ray, A. B. Duah, F. K., Slatkin, D. J. and Schiff, P. L. Jr., J. Nat. Prod. 1984, 47, 179], two C-glycosides, 8-C-β-D-glucopyranosyl-3, 7, 4'-trihydroxy and 3, 7, 3', 4'-tetrahydroxy flavone and 3'-C-β-D-glucopyranosyl -α-hydroxy dihydrochalcone [Bezuidenhoudt, B. C. B., Brandt, E. V., and Ferreira, E. V., Phytochemistry, 1987, 26, 531] from ethyl acetate extract of defatted heartwood.

However, the prior art does not provide any details about the biological activities associated with such chemical constituents. Also prior art discloses only preparation of ether extract, ethyl acetate extract and ethyl acetate soluble fraction of the alcoholic extract but does not disclose any method of preparing water extracts of heartwood of *Pterocarpus marsupium* and attempting to isolate any chemical constituents therefrom.

### Objects of the invention

The main object of the invention is to accordingly prepare water extracts of the heartwood of *Pterocarpus marsupium* and to obtain chemical constituents therefrom.

It is another object of the invention to obtain novel bioactive fractions from water extracts of heartwood of *Pterocarpus marsupium* which are useful in treatment of diabetes.

### Summary of the invention

The above and other objects of the invention are achieved by partitioning an aqueous extract of powdered heartwood of *Pterocarpus marsupium* with different organic solvents. The novel bioactive fraction, 6-hydroxy-2-*p*-hydroxybenzylbenzofuran-7-C-β-D-glucopyranoside is isolated from the polar fraction by choromatographic techniques and is found to show hypoglycaemic activity. There is no disclosure in the prior art of this compound since work had been done in the art on the ether extract, ethyl acetate extract and ethyl acetate soluble fraction of the alcoholic extract.

Accordingly, the present invention provides a novel glucopyranoside 6-hydroxy-2-*p-*hydroxybenzylbenzofuran-7-C-β-D-glucopyranoside of formula **1** where R is H or COCH₃

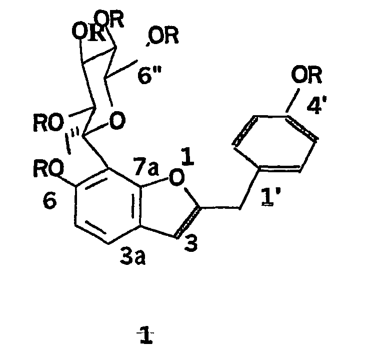

The present invention also provides a process for the isolation of 6-hydroxy-2-*p-*hydroxybenzylbenzofuran-7-C-β-D-glucopyranoside of the formula **1** which comprises:
(a) powdering the heartwood of the plant *Pterocarpus marsupium,*
(b) extracting the powdered plant material with a protic solvent,
(c) concentrating the extract to minimum volume and partitioning with different organic solvents of increasing polarity to remove non-polar components, extracting the aqueous layer with polar solvent, removing the solvent to get the residue.,
(d) isolating 6-hydroxy-2-*p*-hydroxybenzylbenzofuran-7-C-β-D-glucopyranoside from the residue.

In one embodiment of the invention, the protic solvent used for preparing the extract in step (b) is selected from the group consisting of water, methanol, ethanol, propanol, butanol and any mixture thereof

In another embodiment of the invention, the organic solvents used in step (c) comprise solvents of increasing polarity containing 1 to 6 carbon atoms in the molecule.

In another embodiment of the invention, the organic solvents of increasing polarity used in step (c) to remove the non-polar components comprise hexane, chloroform, methanol and ethanol in that order.

In another embodiment of the invention the organic solvents of increasing polarity used to extract the aqueous layer comprise hexane, chloroform, ethyl acetate and methanol in that order.

In another embodiment of the invention the organic solvents of increasing polarity used to extract the aqueous layer comprise hexane, chloroform, ethyl acetate, propanol and n-butanol in that order.

In another embodiment of the invention, the chromatographic methods used for the isolation of 6-hydroxy-2-*p*-hydroxybenzylbenzofuran-7-C-β-D-glucopyranoside is selected from MPLC, HPLC and flash chromatography.

The present invention also provides a pharmaceutical composition containing a pharmaceutically effective amount of 6-hydroxy-2-*p*-hydroxybenzylbenzofuran-7-C-β-D-glucopyranoside of formula 1 in a pharmaceutically acceptable carrier.

In one embodiment of the invention, the amount of 6-hydroxy-2-*p-*hydroxybenzylbenzofuran-7-C-β-D-glucopyranoside in said composition is in the range of 0.5 mg to 15 mg per kg of body weight of the patient.

The invention also relates to a method for the treatment of diabetes comprising administering a pharmaceutically effective amount of 6-hydroxy-2-*p-*hydroxybenzylbenzofuran-7-C-β-D-glucopyranoside to a patient.

In one embodiment of the invention, the amount of 6-hydroxy-2-*p-*hydroxybenzylbenzofuran-7-C-β-D-glucopyranoside in said composition is in the range of 0.5 mg to 15 mg per kg of body weight of the patient.

The present invention also relates to the use of 6-hydroxy-2-*p-*hydroxybenzylbenzofuran-7-C-β-D-glucopyranoside in the preparation of a pharmaceutical composition for the treatment of diabetes.

In one embodiment of the invention, the amount of 6-hydroxy-2-*p-*hydroxybenzylbenzofuran-7-C-β-D-glucopyranoside in said composition is in the range of 0.5 mg to 15 mg per kg of body weight of the patient.

### Detailed description of the invention

The present invention provides a process for the isolation of 6-hydroxy-2-*p-*hydroxybenaylbenzofuran-7-C-β-D-glucopyranoside which comprises:
(a) powdering the heartwood of the plant *Pterocarpus marsupium,*
(b) extracting the powdered plant material so prepared with a protic solvent,
(c) concentrating the aqueous extract to minimum volume and partitioning with organic solvents of increasing polarity to remove non-polar components, extracting the aqueous layer with polar solvent, removing the solvent to get the residue;
(d) isolating the 6-hydroxy-2-p-hydroxybenzylbenzofuran-7-C-β-D-glucopyranoside from residue.

The solvent used for preparing the extract may be water, methanol, ethanol, propanol and butanol and like or their mixtures. The organic solvent used in step (c) to remove the non-polar components is selected from hexane, ethyl acetate, methanol, ethanol, propanol, n-butanol and chloroform. The polar solvent used to extract the aqueous layer is selected from ethyl acetate, propanol, butanol and a mixture thereof The chromatographic methods used for the isolation of methanol, ethanol, propanol may be MPLC, flash chromatography etc.

In the MPLC method the required eluting solvent is pumped through the column and in the flash chromatography solvent is pushed with air pressure. The compound of the invention was recrystallised from a mixture of ethyl acetate and methanol, mp 117 - 118°C, [α]_{D}¹⁹ + 9.15°(MeOH, c, 0.295), showed UV maxima at 242, 253 and 284 nm in methanol. The molecular formula of the compound was established as C₂₁H₂₂O₈ on the basis of strong peak at m/z 402 [M]⁺ in the FAB mass spectrum, together with the support of spectroscopic methods.

The compound 6-hydroxy-2-*p*-hydroxybenzylbenzofuran-7-C-β-D-glucopyranoside was isolated from the n-butanol soluble fraction of the water decoction of the heartwood of *P*. *marsupium* which has shown antidiabetic activity in both humans and animals. There is no disclosure in the prior art of this compound since work had been done in the art on the ether extract, ethyl acetate extract and ethyl acetate soluble fraction of the alcoholic extract.

The process of isolating active principle from *Pterocarpus marsupium* comprises partition of the aqueous extract of powdered heartwood with different organic solvents containing 1-6 carbon atoms in the molecule. 6-hydroxy-2-*p*-hydroxybenzylbenzofuran-7-C-β-D-glucopyranoside of formula 1 is isolated from polar fraction by applying modem chromatographic techniques such as medium pressure liquid chromatography (MPLC), high pressure liquid chromatography (HPLC) and flash chromatography using silica gel (230 - 400 mesh) and shows hypoglycaemic activity.

The 6-hydroxy-2-*p*-hydroxybenzylbenzofuran-7-C-β-D-glucopyranoside isolated from *Pterocarpus marsupium* possesses anti-diabetic activity.

The chromatographic methods used for the isolation of 6-hydroxy-2-*p-*hydroxybenzylbenzofuran-7-C-β-D-glucopyranoside may be MPLC, flash chromatography etc. In the MPLC method the solvent is pumped through the column and in the flash chromatography is pushed with air pressure. The IR spectrum revealed absorptions at 3300 for hydroxyls, 1600, 1584, 1512 cm⁻¹ for aromatic ring. The ¹H and ¹³C NMR spectra exhibited two sets of multiplets for aromatic protons centered at δ 7.18 (H-5,3',5') and 6.70 (H-4, 2', 6'), one furan proton singled at δ 6.27 (H-3), δC 102.9, a singlet for one benzylic methylene group at δ 3.65, δC 34.2 and multiplet at δ 3.00 - 5.00 for sugar protons. The spectral data suggest that the compound of the invention is a benzofuran C-glucoside containing one phenolic hydroxy group in ring -C. On acetylation the compound of the invention furnished hexa-acetate where in formula 1 R is acetyl, recrystallised from methanol, mp 80-81°C, [α]_{D}¹⁹ -85.40° (CHCl₃, c, 0.185), showed UV maxima at 248, 252, 278, 286 nm in chloroform, IR in KBr 1725, 1600, 1580, 1385 cm⁻¹, the molecular formula of the hexaacetate being C₃₃H₃₄O₁₄, m/z 655[M+1}⁺. The ¹H and ¹³C NMR spectra indicated the presence of four singlets for sugar acetate groups at δ 2.17, 2.16, 2.09, and 2.08, two singlets for aromatic acetate groups at δ 2.42, and 2.35, one singlet for bynzylic methylene group at δ 4.16, δC 34.3, one singlet for furan proton at δ 6.38, δC 103.4, two *ortho* coupled aromatic protons at δ 7.46 (1H, d, J = 8.4 Hz), and 6.92 (1H, d, J = 8.4 Hz), one A₂B₂ aromatic system at δ 7.36 (2H, d, J = 8.3 Hz) and 7.10 (2H, d, J = 8.3 Hz). The anomeric proton of sugar appeared at δ 5.02 (1H, d, J = 9.9 Hz), δC 74.5, indicating it to have the β-configuration on the basis of chemical shift and coupling constant [Roberts J.D., Weigert, F.J., Kroschwitz, J.I. and Reich, H.J., J. Am. Chem.Soc., 1970, 92, 1338]. Further methine protons of sugar appeared at δ 5.73 (1H, d, J = 9.3 Hz), 5.42 (1H, d, J = 9.2 Hz) and 5.28 (1H, d, J = 9.4 Hz). Coupling constants for the methine protons H-1" to H-5" of the hexose showed an all *trans-*axial relationship and together with the methylene (H-6") resonances confirmed the identity of sugar as β-D-glucose. Further the carbon chemical shifts of the glucose moiety were congruent with those of C-β-D-glucopyranosyl residue [Ikeya, Y., Sugama, K., and Maruno, M., Chemistry and Pharmacology Bulletin, 1994, 42, 2305] and HMBC spectra indicated it is linked to aglycone at C-7. On the basis of the above spectral data the structure of the compound was established as 6-hydroxy-2-p-hydroxybenzylbenzofuran-7-C-β-D-glucopyranoside of the formula 1 where R is hydrogen.

The compound was evaluated for hypoglycaemic activity in 18 hour fasted Wistar rats. In a dose of 15 mg/kg p.o., hypoglycaemic effect was recorded in all the treated rats. The mean fall recorded was 24 mg/100 ml blood, from an initial mean of 91 to mean of 67 mg/100 ml blood. As compared to this, conventional hypoglycaemic agents such as chlorpropamide used as a positive control showed mean fall of 18 mg/100 ml of blood.

### Example 1

The powdered heartwood of *Pterocarpus marsupium* (1kg) was percolated with 80% aqueous ethanol (3x3 lits.) for a period of 48 hours. The resultant concentrate was partitioned with hexane, chloroform, propanol and butanol in that order. The polar extract was subjected to MPLC using silica gel (100 - 200 mesh) for gross fractions with hexane, chloroform, methanol, ethanol in that order. The active compound was purified by repeated MPLC and flash chromatography over silca gel (230 - 400 mesh) using CHCl₃ - MeOH (9:1) as solvent, to furnish 6-hydroxy-2-p-hydroxybenzylbenzofuran-7-C-β-D-glucopyranoside of the formula 1, (yield 0.031 - 0.041%), mp. 117 -118°C, [α]_{D}¹⁹ + 9.15° (MeOH, c, 0.295), and hexaacetate of compound of formula 1 where R is acetyl recrystallised from methanol, mp 80-81°C, [α]_{D}¹⁹ -85.40° (CHCl₃, c, 0.185).

### Example 2

The heartwood of *Pterocarpus marsupium* was extracted with hot water for a period of 4 hours. The resultant concentrate was partitioned between hexane, chloroform, propanol and butanol in that order. The polar extract so obtained was subjected to flash chromatography employing silica gel (100 - 200 mesh) using hexane, chloroform, ethylacetate and methanol as solvent system to afford 6-hydroxy-2-p-hydroxybenzylbenzofuran-7-C-β-D-glucopyranoside rich fraction, which on repeated chromatography over silica gel (230 - 400 mesh) using EtOAc - MeOH (19:1) as solvent, furnished 6-hydroxy-2-p-hydroxybenzylbenzofuran-7-C-β-D-glucopyranoside of the formula 1, (yield 0.032 - 0.043%), mp. 117 -118°C, [α]_{D}¹⁹ + 9.15° (MeOH, c, 0.295), and hexaacetate of compound of formula 1 where R is acetyl recrystallised from methanol, mp 80-81°C, [α]_{D}¹⁹ 85.40° (CHCl₃, c, 0.185).

### Example 3

The heartwood of *Pterocarpus marsupium* was boiled with water (16 times) till 1/4 volume of water is left, filtered, concentrated and partitioned between hexane, chloroform, ethyl acetate, propanol and n-butanol in that order. The polar extract obtained was subjected to column chromatography employing silica gel (60-120 mesh) using hexane, chloroform, ethyl acetate and methanol as solvent system to afford 6-hydroxy-2-p-hydroxybenzylbenzofuran-7-C-β-D-glucopyranoside rich fraction. The 6-hydroxy-2-p-hydroxybenzylbenzofuran-7-C-β-D-glucopyranoside rich fraction on repeated column chromatography over silica gel (100-200 mesh) using mixture of ethyl acetate - acetone (7:3), furnished 6-hydroxy-2-p-hydroxybenzylbenzofuran-7-C-β-D-glucopyranoside of the formula 1 (yield 0.031 - 0.044%), mp. 117 -118°C, [α]_{D}¹⁹ + 9.15° (MeOH, c, 0.295), hexaacetate of compound of formula 1 where R is acetyl, recrystallised from methanol, mp 80-81°C, [α]_{D}¹⁹ -85.40° (CHCl₃, c, 0.185)..

### Advantages:

1. The compound obtained 6-hydroxy-2-p-hydroxybenzylbenzofuran-7-C-β-D-glucopyranoside is a novel molecule with antidiabetic activity.
2. The method of isolation of 6-hydroxy-2-p-hydroxybenzylbenzofuran-7-C-β-D-glucopyranoside is comparatively simple.

## Claims

1. A novel glucopyranoside 6-hydroxy-2-p-hydroxybenzylbenzofuran-7-C-β-D-glucopyranoside of the formula **1** 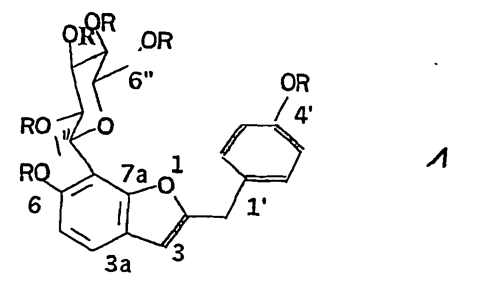 where R is H or COCH₃

2. Process for the isolation of 6-hydroxy-2-p-hydroxybenzylbenzoiuran-7-C-β-D-glucopyranoside of the formula 1 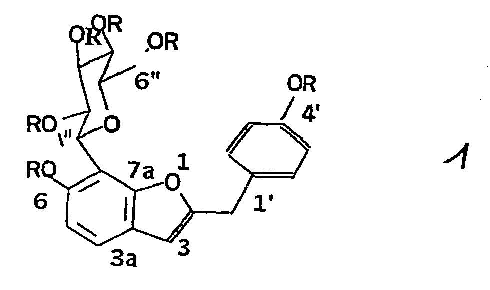 where R is H or COCH₃ which comprises:
(a) powdering the heartwood of the plant *Pterocarpus marsupium,*
(b) extracting the powdered plant material with a protic solvent,
(c) concentrating the extract to minimum volume and partitioning with different organic solvents of increasing polarity to remove non-polar components, extracting the aqueous layer with polar solvent, removing the solvent to get the residue.,
(d) isolating the 6-hydroxy-2-p-hydroxybenzylbenzofuran-7-C-β-D-glucopyranoside from the residue.

3. Process as claimed in claim 2 wherein the protic solvent used for preparing the extract in step (b) is selected from the group consisting of water, methanol, ethanol, propanol, butanol and any mixture thereof

4. Process as claimed in claim 2 wherein the organic solvents used in step (c) comprise solvents of increasing polarity containing 1 to 6 carbon atoms in the molecule.

5. Process as claimed in claim 2 wherein the organic solvents of increasing polarity used in step (c) to remove the non-polar components comprise hexane, chloroform, methanol and ethanol in that order.

6. Process as claimed in claim 2 wherein the organic solvents of increasing polarity used to extract the aqueous layer comprise hexane, chloroform, ethyl acetate and methanol in that order.

7. Process as claimed in claim 2 wherein the organic solvents of increasing polarity used to extract the aqueous layer comprise hexane, chloroform, ethyl acetate, propanol and n-butanol in that order.

8. Process as claimed in claim 2 wherein the chromatographic methods used for the isolation of 6-hydroxy-2-*p*-hydroxybenzylbenzofuran-7-C-β-D-glucopyranoside is selected from MPLC, HPLC and flash chromatography.

9. Pharmaceutical composition containing a pharmaceutically effective amount of 6-hydroxy-2-*p*-hydroxybenzylbenzofuran-7-C-β-D-glucopyranoside of formula 1 in a pharmaceutically acceptable carrier.

10. Composition as claimed in claim 9 wherein the amount of 6-hydroxy-2-*p-*hydroxybenzylbenzofuran-7-C-β-D-glucopyranoside in said composition is in the range of 0.5 mg to 15 mg per kg of body weight of the patient.

11. Use of 6-hydroxy-2-*p*-hydroxybenzylbenzofuran-7-C-β-D-glucopyranoside in the preparation of a pharmaceutical composition for the treatment of diabetes.

12. Use as claimed in claim 11 wherein the amount of 6-hydroxy-2-*p-*hydroxybenzylbenzofuran-7-C-β-D-glucopyranoside in said composition is in the range of 0.5 mg to 15 mg per kg of body weight of the patient.

## Patentansprüche

1. Neues Glucopyranosid 6-Hydroxy-2-p-hydroxybenzylbenzofuran-7-C-β-D-glucopyranosid der Formel 1 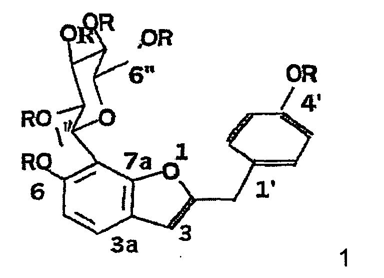 in der R für H oder COCH₃ steht.

2. Verfahren zur Isolierung von 6-Hydroxy-2-p-hydroxybenzylbenzofuran-7-C-β-D-glucopyranosid der Formel 1 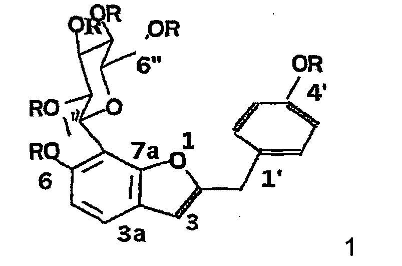 in der R für H oder COCH₃ steht,
welches umfasst:
(a) Pulverisieren des Hartholzes der Pflanze *Pterocarpus marsupium,*
(b) Extrahieren des pulverförmigen Pflanzenmaterials mit einem protischen Lösungsmittel,
(c) Konzentrieren des Extrakts auf ein minimales Volumen und Verteilen mit verschiedenen organischen Lösungsmitteln zunehmender Polarität, um unpolare Komponenten zu entfernen, Extrahieren der wässrigen Schicht mit einem polaren Lösungsmittel, Entfernen des Lösungsmittels, um den Rückstand zu erhalten,
(d) Isolieren des 6-Hydroxy-2-p-hydroxybenzylbenzofuran-7-C-β-D-glucopyranosids aus dem Rückstand.

3. Verfahren nach Anspruch 2, bei dem das protische Lösungsmittel, das zur Herstellung des Extrakts im Schritt (b) verwendet wird, ausgewählt ist aus der Gruppe bestehend aus Wasser, Methanol, Ethanol, Propanol, Butanol und irgendeiner Mischung derselben.

4. Verfahren nach Anspruch 2, bei dem die im Schritt (c) verwendeten organischen Lösungsmittel Lösungsmittel mit zunehmender Polarität umfassen, die 1 bis 6 Kohlenstoffatome im Molekül enthalten.

5. Verfahren nach Anspruch 2, bei dem die organischen Lösungsmittel mit zunehmender Polarität, die im Schritt (c) verwendet werden, um unpolare Komponenten zu entfernen, Hexan, Chloroform, Methanol und Ethanol in dieser Reihenfolge umfassen.

6. Verfahren nach Anspruch 2, bei dem die organischen Lösungsmittel mit zunehmender Polarität, die zum Extrahieren der wässrigen Schicht verwendet werden, Hexan, Chloroform, Ethylacetat und Methanol in dieser Reihenfolge umfassen.

7. Verfahren nach Anspruch 2, bei dem die organischen Lösungsmittel mit zunehmender Polarität, die zum Extrahieren der wässrigen Schicht verwendet werden, Hexan, Chloroform, Ethylacetat, Propanol und n-Butanol in dieser Reihenfolge umfassen.

8. Verfahren nach Anspruch 2, bei dem die chromatographischen Verfahren, die zur Isolierung von 6-Hydroxy-2-p-hydroxybenzylbenzofuran-7-C-β-D-glucopyranosid verwendet werden, aus MPLC, HPLC und FlashChromatographie ausgewählt sind.

9. Pharmazeutische Zusammensetzung, enthaltend eine pharmazeutisch wirksame Menge von 6-Hydroxy-2-p-hydroxybenzylbenzofuran-7-C-β-D-glucopyranosid der Formel 1 in einem pharmazeutisch annehmbaren Träger.

10. Zusammensetzung nach Anspruch 9, in der die Menge an 6-Hydroxy-2-p-hydroxybenzylbenzofuran-7-C-β-D-glucopyranosid in der Zusammensetzung im Bereich von 0,5 mg bis 15 mg pro kg Körpergewicht des Patienten liegt.

11. Verwendung von 6-Hydroxy-2-p-hydroxybenzylbenzofuran-7-C-β-D-glucopyranosid bei der Herstellung einer pharmazeutischen Zusammensetzung zur Behandlung von Diabetes.

12. Verwendung nach Anspruch 11, bei der die Menge von 6-Hydroxy-2-p-hydroxybenzylbenzofuran-7-C-β-D-glucopyranosid in der Zusammensetzung im Bereich von 0,5 mg bis 15 mg pro kg Körpergewicht des Patienten liegt.

## Revendications

1. Nouveau glucopyranoside, le 6-hydroxy-2-p-hydroxybenzylbenzofurane-7-C-β-D-glucopyranoside de formule 1 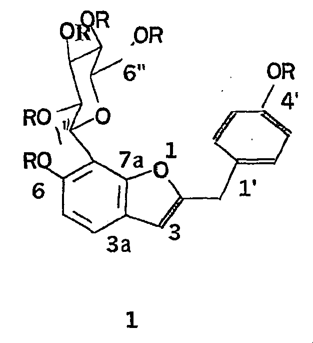 où R représente H ou COCH₃.

2. Procédé d'isolement du 6-hydroxy-2-p-hydroxybenzylbenzofurane-7-C-β-D-glucopyranoside de formule 1 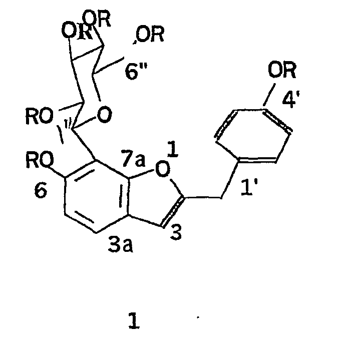 où R représente H ou COCH₃
qui comprend :
(a) la pulvérisation du duramen de la plante *Pterocarpus marsupium,*
(b) l'extraction du matériau végétal pulvérisé avec un solvant protique,
(c) la concentration de l'extrait jusqu'à un volume minimal et le partage avec différents solvants organiques de polarité croissante pour éliminer les composants non polaires, l'extraction de la phase aqueuse avec un solvant polaire, l'élimination du solvant pour obtenir le résidu,
(d) l'isolement du 6-hydroxy-2-p-hydroxybenzylbenzofurane-7-C-β-D-glucopyranoside à partir du résidu.

3. Procédé selon la revendication 2, dans lequel le solvant protique utilisé pour préparer l'extrait dans l'étape (b) est choisi dans le groupe constitué d'eau, de méthanol, d'éthanol, de propanol, de butanol et de tout mélange de ceux-ci.

4. Procédé selon la revendication 2, dans lequel les solvants organiques utilisés dans l'étape (c) comprennent des solvants de polarité croissante contenant 1 à 6 atomes de carbone dans la molécule.

5. Procédé selon la revendication 2, dans lequel les solvants organiques de polarité croissante utilisés dans l'étape (c) pour éliminer les composants non polaires comprennent l'hexane, le chloroforme, le méthanol et l'éthanol dans cet ordre.

6. Procédé selon la revendication 2, dans lequel les solvants organiques de polarité croissante utilisés pour extraire la couche aqueuse comprennent l'hexane, le chloroforme, l'acétate d'éthyle et le méthanol dans cet ordre.

7. Procédé selon la revendication 2, dans lequel les solvants organiques de polarité croissante utilisés pour extraire la couche aqueuse comprennent l'hexane, le chloroforme, l'acétate d'éthyle, le propanol et le n-butanol dans cet ordre.

8. Procédé selon la revendication 2, dans lequel les procédés chromatographiques utilisés pour l'isolement du 6-hydroxy-2-p-hydroxybenzylbenzofurane-7-C-β-D-glucopyranoside sont choisis parmi la CLMP, la CLHP et la chromatographie flash.

9. Composition pharmaceutique contenant une quantité pharmaceutiquement efficace de 6-hydroxy-2-*p-*hydroxy-benzylbenzofurane-7-C-β-D-glucopyranoside de formule 1 dans un support pharmaceutiquement acceptable.

10. Composition selon la revendication 9, dans laquelle la quantité de 6-hydroxy-2-p-hydroxybenzylbenzofurane-7-C-β-D-glucopyranoside dans ladite composition se situe dans la plage de 0,5 mg à 15 mg par kg de poids corporel du patient.

11. Utilisation de 6-hydroxy-2-p-hydroxybenzylbenzofurane-7-C-β-D-glucopyranoside dans la préparation d'une composition pharmaceutique destinée au traitement du diabète.

12. Utilisation selon la revendication 11, où la quantité de 6-hydroxy-2-*p*-hydroxybenzylbenzofurane-7-C-β-D-glucopyranoside dans ladite composition se situe dans la plage de 0,5 mg à 15 mg par kg de poids corporel du patient.
